# EUROPEAN PATENT SPECIFICATION

(11) **EP 0 704 196 B1**
(45) Date of publication and mention of the grant of the patent: **14.07.1999**
(21) Application number: 94115429.6
(22) Date of filing: 30.09.1994
(51) Int. Cl.: A61F 13/58, C09J 7/04

(54) **Fastening tape for a sanitary article, particularly disposable diaper**
Befestigungsband für einen Sanitärartikel, insbesondere Wegwerfwindel
Bande de fixation pour un article sanitaire, en particulier pour une couche à jeter

(43) Date of publication of application: 03.04.1996
(73) Proprietor: THE PROCTER & GAMBLE COMPANY, Cincinnati, Ohio 45202 (US)
(72) Inventor: Schmitt, Achim c/o Procter & Gamble GmbH, D-65824 Schwalbach am Taunus (DE); Hodgetts, Jonathon c/o Procter & Gamble GmbH, D-65824 Schwalbach am Taunus (DE)
(74) Representative: TER MEER STEINMEISTER & PARTNER GbR

(56) References cited:
- EP-A- 0 191 355
- EP-A- 0 249 073
- EP-A- 0 338 680
- EP-A- 0 487 758
- US-A- 4 063 559
- US-A- 4 209 016

## Description

The invention relates to a fastening tape for a sanitary article, particularly disposable diaper, for fastening of the article on the body of a person, the fastening tape being attached to the article at one of its end portions and being provided with a fastening means on one surface of the other end portion, a stretchable elastic portion being provided between the end portions, a tape section of a stretchable elastic material being secured to the fasting tape at least at both endsthereof, to bridge a section of the fastening tape which is longer than the stretchable elastic tape section.

A fasting tape of this kind is disclosed by the US-A 4 209 016. This document describes a disposable diaper having a fasting tape a portion of which is folded and bridged by an elastic tape section.

Conventional sanitary articles like disposable diapers are provided with fastening tapes attached to the diaper and provided with a layer of pressure-sensitive adhesive at the end portion thereof. The tapes are used to close the diaper round the wearer's body and to fasten the diaper on the body. Among these fastening tapes, there have also been elastically stretchable tapes to improve the fit and the comfort of the articles.

However, if the part of the tapes covered by the adhesive layer is stretchable as disclosed by the US-A 4 063 559 or the EP-A1 0 191 355, the tape may be inadvertently released by peeling off under a predetermined stress. Other conventional stretchable fastening tapes as disclosed by the EP-A2 0 249 073 or the EP-A1 0 487 758 consist of stretchable and non-stretchable portions being connected in the longitudinal direction end to end or by overlapping. In these cases, providing of a reliable connection between the portions under industrial conditions with acceptable costs has not yet been possible.

On the other hand, it is not easy to exactly achieve a predetermined elasticity of the tapes. If the forces necessary to stretch the tape exceed a certain limit, there will be no effect and the wearing comfort will not be improved. On the other hand, if the tape is too soft, over-stretching of the tape may occur by which the diaper may get lose or even open.

Thus it is the main object of the invention to provide a fastening tape for a sanitary article useful for the wearing comfort of the article by a comparably soft resiliency without the risk of over-stretching of the tape.

Moreover, it is an object of the invention to provide a tape of the above kind which can be easily manufactured at low costs, and a method of producing the tape accordingly.

To comply with this object, a fastening tape according to the present invention is characterized in that a section of the fasting tape being longer than the stretchable elastic tape section is provided with zigzag-shaped folding extending laterally with respect to the length of the fasting tape.

Thus the invention provides a combination of an elastic tape, to improve wearing comfort of the article in question, and a non-elastic, tape which has a kind of stop function determining the maximum extension of the elastic section to avoid rupture thereof.

The tape section of stretchable elastic material may be secured to the fastening tape at the ends of the elastic material only or along the whole length of the elastic tape or in intervals. Securing the elastic tape in intervals will be particularly suitable in case the section of the fastening tape bridged by the elastic tape achieves a zigzag-folding by a suitable die, particularly by ringrolling. In this case, the vallies of the zigzag-shape may be secured to the elastic tape. Securing the tapes in intervals may be realized by an adhesive or also by different types of welding methods.

In any case, the length of the fastening tape bridged by the elastic tape must be longer than the elastic tape in the relaxed length. In this position the foldings will appear and will make sure that the fastening tape does not deform away from the elastic tape. On the other hand, if the elastic tape is stretched into a predetermined position, the fastening tape will be extended to the straight position thereof where the zigzag-form disappears.

Preferably, foldings are formed in the portion of the fastening tape bridged by the elastic tape section which disappear more and more when the elastic tape section is stretched.

If the laminate formed by the fastening tape and the elastic tape section is ringrolled or deformed by any other pair of dies having a zigzag-shaped gab, the laminate will stretch. After being removed from the dies, the elastic tape will return to its non-stretched position while the zigzag-shaped deformation of the fastening tape will remain. Another possibility to obtain the combination of the fastening tape and the elastic tape would be to secure the elastic tape to the fastening tape in a pre-stressed condition. In this case, when the elastic tape section is released, the corresponding part of the fastening tape will be longer than the elastic tape and will deform, for example in a zig-zag-shape in case both tapes are connected in intervals. Both possibilities, i.e. a zig-zag die deformation and securing the elastic tape section in a pre-stressed condition, may be combined with each other.

The material of the stretchable elastic tape section may be natural rubber or synthetic rubber or any comparable elastomeric material. According to tests the Inventor has made, a stretchability of 2 mm/N appeared to be acceptable.

The material of the fastening tape is more or less non-stretchable and non-elastic under the conditions the fastening tape is used in the present context. The material thereof should be deformable as the fastening tapes used for applicants present products, for instance diapers sold under the trademark Pampers.

Embodiments of the invention will be described with reference to the enclosed drawings which are not intended to limit the scope of the invention.
- Fig. 1: is a cross-section of the edge portion of a diaper being provided with a fastening tape according to the invention;
- Figs. 2 to 4: are corresponding longitudinal sections of the fastening tape according to Fig. 1 illustrating the method for producing an elastically stretchable portion of the tape;
- Figs. 5 to 7: are top views of the tape corresponding the cross-sections according to Figs. 2 to 4;
- Fig. 8: is a perspective view, in larger scale, of a ringrolling step for forming the elastically stretchable portion of the tape;
- Fig. 9: is a top view on the web achieved by ringrolling.

In the following description of embodiments, a diaper will be used as an example for a sanitary article according to the invention. It should be noted that it is not intended to limit the use of the fastening tape according to the invention to diapers. The fastening tape according to the invention is applicable to other disposable sanitary articles which are to be attached to the body of the wearer as well.

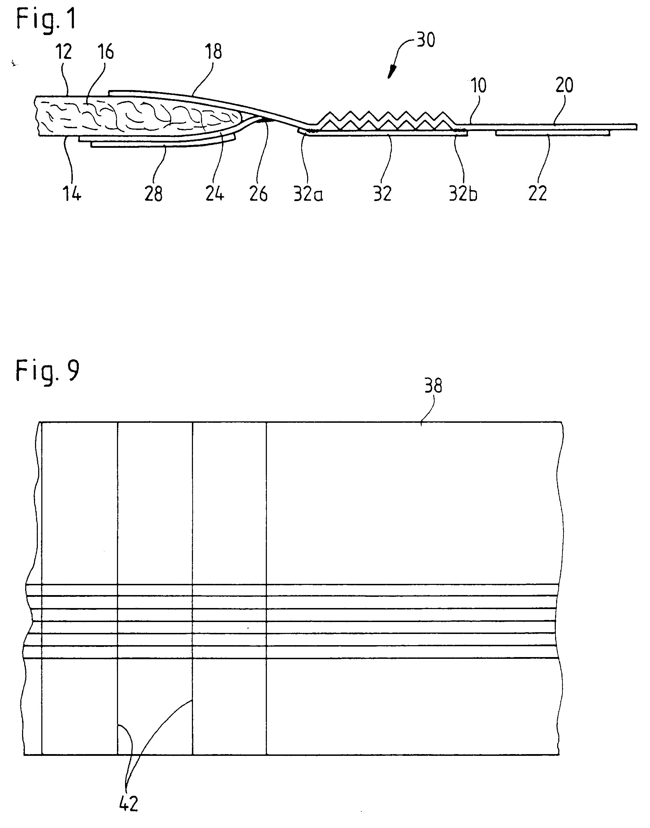

Fig. 1 is a cross-section through the edge of a diaper being provided with a fastening tape 10 according to the invention. The diaper comprises a liquid-impermeable backsheet 12, a liquid-permeable topsheet 14 to face the skin of the wearer and an absorbent core 16 between both.

An end portion 18 of the fastening tape 10 is adhered to the edge portion of the backsheet 12, usually by a hot melt adhesive, while the other end portion 20 of the fastening tape on the right side in Fig. 1 is provided with an adhesive layer 22 of a pressure-sensitive adhesive for closing the diaper in the well-known way. Another adhesive tape 24 is adhered to the edge portion of the topsheet 14 and provided with a projecting end 26 adhered to the lower surface of the fastening tape 10 in Fig. 1. The lower surface of the adhesive tape 24 is covered by a release layer 28 to which the adhesive layer 22 at the end portion 20 of the fastening tape is preliminarily attached before fixing the diaper on a wearer.

A diaper having fastening tapes of this kind is well-known in the art and does not require any further description.

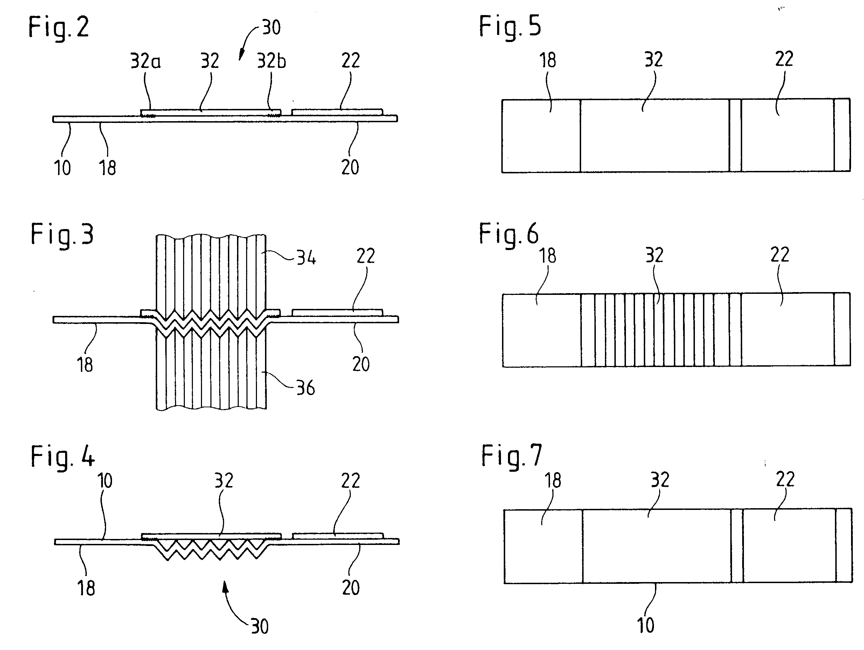

According to the invention, the fastening tape 10 comprises an elastically stretchable center portion 30 between the end portions 18,20. The center portion 30 will be described in detail with reference to Fig. 1 in connection with Figs. 2 to 7. Figs. 2 to 4 show the fastening tape in an upside down orientation compared with Fig. 1. The same reference numerals have been used as in Fig. 1 to designate identical or corresponding members.

The elastically stretchable, central portion 30 is formed by a laminate consisting of the fastening tape 10 and a tape section 32 of an elastic material adhered to the fastening tape with its end portions 32a,32b, to bridge the fastening tape 10 over a certain length thereof. The corresponding top view is shown in Fig. 5.

As shown in Figs. 3 and 6, the laminate consisting of the central portion of the fastening tape 10 and the tape section 32 of elastic material is passed through the gap of a pair of two meshing rolls having saw-tooth shaped corresponding surfaces the ridges of which extend circumferentially. This method is frequently designated as "ringrolling".

If the laminate formed by the fastening tape 10 and the elastic tape section 32 is moved through the gap of these rolls which have been shown in part in Fig. 3 and designated by 34 and 36, both of the layers 10,32 are stretched zigzag-wise. Since the tape section 32 is elastic, the stretch will be recovered after leaving the rolls 34,36, while the material of the fastening tape 10 is not elastic and thus the fastening tape will remain fan-folded in the portion 30, as shown in Fig. 4.

Thus, if a stretching force is exerted to the portion 30, the tape section 32 of elastic material will elastically stretch while the corresponding portion of the fastening tape 10 is extended to a straight form in which no further extension of the elastically stretchable portion 30 is possible. The portion of the fastening tape 10 bridged by the elastic tape section 32 thus forms a kind of stop determining the end position of the stretch of the elastic tape section.

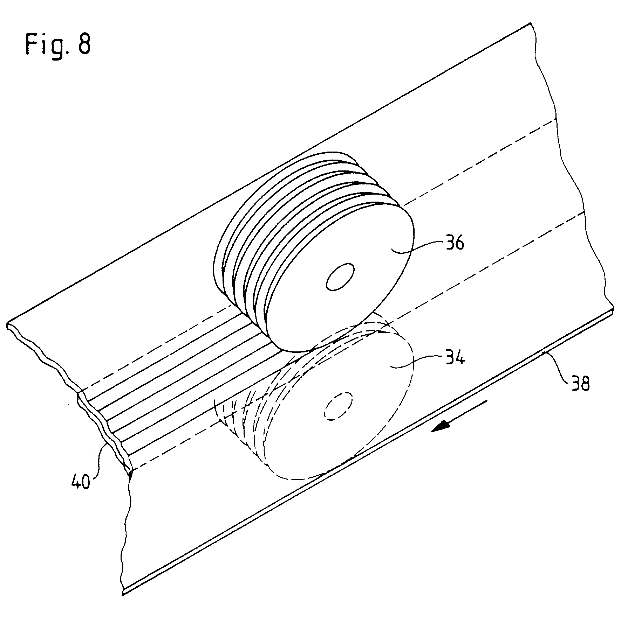

The fastening tape 10 may be cut from an endless web 38 shown in a perspective view in Figs. 8 and 9.

According to Figs. 8 and 9, a web 38 having a width corresponding to the length of the fastening tape 10, is passed lengthwise through the pair of intermeshing rolls 34,36. A tape 40 of an elastic material is adhered to the lower surface of the web 38 which is not seen in Figs. 8 and 9. This tape 40 covers the lower surface of the web 38 over a width slightly extending beyond the track of the lower roll 34 on both sides, as may be taken from Fig. 8. When passing the gap of the rolls 34,36, the laminate consisting of the web 38 and the tape 40 is experiencing the folding process described in connection with Figs. 3 and 6. Before or after this ringrolling step, a strip of a pressure sensitive adhesive may be applied to the web 38 to the surface not visible in Figs. 8 and 9, to form the adhesive layer 22 on the lower side of the fastening tape 10 shown in Fig. 1. Finally, the web 38 is cut by cutting lines 42 as shown in Fig. 9, to form fastening tapes 10 according to Fig. 1.

## Claims

1. Fastening tape for a sanitary article, particularly disposable diaper, for fastening of the article on the body of a person, the fastening tape being attached to the article at one of its end portions (18) and being provided with a fastening means (22) on one surface of the other end portion (20), a stretchable elastic portion (30) being provided between the end portions, a tape section (32) of a stretchable elastic material being secured to the fasting tape (10) at least at both ends (32a,32b) thereof, to bridge a section of the fastening tape (10) which is longer than the stretchable elastic tape section (32), **characterized** in that said section of the fasting tape (10) being longer than the stretchable elastic tape section (32) is provided with zigzag-shaped folding extending laterally with respect to the length of the fasting tape.

2. Fastening tape according to claim 1, **characterized** in that the ends (32a,32b) of the elastic tape section (32) are secured to the fastening tape (10) by an adhesive.

3. Fastening tape according to claims 1, **characterized** in that the ends (32a,32b) of the elastic tape section (32) are secured to the fastening tape (10) by welding including heat welding and ultrasonic welding.

4. Fastening tape according to one of claims 1 to 3, **characterized** in that the fastening means (22) is formed by an adhesive layer on one surface of the other end portion (20) of the fastening tape.

5. Fastening tape according to one of claims 1 to 3, **characterized** in that the fastening means (22) is formed by mechanical fastening means including Velcro tapes, hooks, buttons and the like.

6. Fastening tape as claimed in one of claims 1 to 5, **characterized** in that the tape section (32) of a stretchable elastic material is secured to the fastening tape (10) over the whole length of the tape section of elastic material.

7. Fastening tape as claimed in one of claims 1 to 5, **characterized** in that the tape section (32) of a stretchable elastic material is secured to the fastening tape (10) in intervals between the ends (32a,32b) of the tape of elastic material.

8. Method for manufacturing a fastening tape according to one of claims 1 to 7, **characterized** in that the elastic tape section (32) is secured at least at the ends (32a,32b) thereof to the fastening tape to bridge a central portion thereof, and that the laminate comprising the fastening tape and the elastic tape section is inserted into a zigzag-shaped gap of two dies.

9. Method for manufacturing a fastening tape according to claim 8, **characterized** in that an elongated web (38) for forming the fastening tapes is provided with a tape (40) of an elastic material which is secured to the web in the longitudinal direction thereof (38) by adhering at least the lateral edges of the tape to one surface of the web (38), and that the strip of the web forming a laminate of the web and the elastic tape is ringrolled, after which fastening tapes are formed by cutting the web laterally.

10. Method as claimed in claim 8 or 9, **characterized** in that the tape section of stretchable elastic material is secured to the fastening tape over the whole length of the tape section of elastic material.

11. Method as claimed in claim 8 or 9, **characterized** in that the tape section of stretchable elastic material is secured to the fastening tape in intervals between the both ends (32a,32b) of the elastic material.

12. Method as claimed in one of claims 8 to 11, **characterized** in that the dies for deforming the laminate of the fastening tape and the elastic tape section are formed by ringrolls.

## Patentansprüche

1. Befestigungsband für einen Sanitärartikel, insbesondere eine Wegwerfwindel, zur Befestigung des Artikels am Körper einer Person, welches Befestigungsband an einem seiner Endbereiche (18) an dem Artikel befestigt ist und auf einer Oberfläche des anderen Endbereichs (20) mit einem Befestigungsmittel (22) versehen ist, wobei ein dehnbarer elastischer Bereich (30) zwischen den Endbereichen vorgesehen ist, und ein Bandabschnitt (32) aus dehnbarem elastischen Material zur Überbrückung eines Abschnitts des Befestigungsbands (10), der länger ist als der dehnbare elastische Bandabschnitt (32), am Befestigungsband (10) zumindest an dessen beiden Enden (32a,32b) befestigt ist, dadurch **gekennzeichnet**, daß der Abschnitt des Befestigungsbands (10), der länger ist als der dehnbare elastische Bandabschnitt (32), mit einer Zickzack-förmigen Faltung versehen ist, die sich bezogen auf die Länge des Befestigungsbands in der Breite erstreckt.

2. Befestigungsband gemäß Anspruch 1, dadurch **gekennzeichnet**, daß die Enden (32a,32b) des elastischen Bandabschnitts (32) durch einen Klebstoff am Befestigungsband (10) befestigt sind.

3. Befestigungsband gemäß Anspruch 1, dadurch **gekennzeichnet**, daß die Enden (32a,32b) des elastischen Bandabschnitts (32) durch Schweißen, einschließlich Heißschweißen und Ultraschallschweißen, am Befestigungsband (10) befestigt sind.

4. Befestigungsband gemäß einem der Ansprüche 1 bis 3, dadurch **gekennzeichnet**, daß das Befestigungsmittel (22) durch eine Kiebstoffschicht auf einer Oberfläche des anderen Endbereichs (20) des Befestigungsbands gebildet wird.

5. Befestigungsband gemäß einem der Ansprüche 1 bis 3, dadurch **gekennzeichnet**, daß das Befestigungsmittel (22) durch mechanische Befestigungsmittel einschließlich Klettbändern, Haken, Knöpfe oder dergleichen gebildet wird.

6. Befestigungsband gemäß einem der Ansprüche 1 bis 5, dadurch **gekennzeichnet**, daß der Bandabschnitt (32) aus dehnbarem elastischen Material über die gesamte Länge des Bandabschnitts aus elastischem Material am Befestigungsband (10) befestigt ist.

7. Befestigungsband gemäß einem der Ansprüche 1 bis 5, dadurch **gekennzeichnet**, daß der Bandabschnitt (32) aus dehnbarem elastischen Material in Abständen zwischen den Enden (32a,32b) des Bands aus elastischem Material am Befestigungsband (10) befestigt ist.

8. Verfahren zur Herstellung eines Befestigungsbands gemäß einem der Ansprüche 1 bis 7, dadurch **gekennzeichnet**, daß der elastische Bandabschnitt (32) zumindest an seinen Enden (32a,32b) am Befestigungsband zur Überbrückung von dessen mittlerem Bereich befestigt ist, und daß das Schichtgebilde mit dem Befestigungsband und dem elastischen Bandabschnitt in einen Zickzack-förmigen Zwischenraum zwischen zwei Formen eingeführt wird.

9. Verfahren zur Herstellung eines Befestigungsbands gemäß Anspruch 8, dadurch **gekennzeichnet**, daß eine Gewebebahn (38) zur Bildung der Befestigungsbänder mit einem Band (40) aus einem elastischen Material versehen ist, das auf der Bahn in deren Längsrichtung (38) durch Kleben zumindest der Seitenkanten des Bands auf einer Oberfläche der Bahn (38) befestigt ist, und daß der Streifen der Gewebebahn, der ein Schichtgebilde der Gewebebahn und des elastischen Bands bildet, ringgewalzt wird, und daß danach Befestigungsbänder durch Schneiden der Gewebebahn in Breitenrichtung gebildet werden.

10. Verfahren gemäß Anspruch 8 oder 9, dadurch **gekennzeichnet**, daß der Bandabschnitt aus dehnbarem elastischen Material über die gesamte Länge des Bandabschnitts aus elastischem Material am Befestigungsband befestigt ist.

11. Verfahren gemäß Anspruch 8 oder 9, dadurch **gekennzeichnet**, daß der Bandabschnitt aus dehnbarem elastischen Material am Befestigungsband in Abständen zwischen den beiden Enden (32a,32b) des elastischen Materials befestigt ist.

12. Verfahren gemäß einem der Ansprüche 8 bis 11, dadurch **gekennzeichnet**, daß die Formen zur Formung des Schichtgebildes aus dem Befestigungsband und dem elastischen Bandabschnitt durch Ringwalzen gebildet werden.

## Revendications

1. Ruban de fixation pour un article hygiénique, en particulier une couche jetable, pour fixer l'article sur le corps d'une personne, le ruban de fixation étant fixé à l'article à une de ses parties d'extrémité (18) et étant pourvu d'un moyen de fixation (22) sur une surface de l'autre partie d'extrémité (20), une partie élastique étirable (30) étant prévue entre les parties d'extrémité, une zone de ruban (32) réalisée avec un matériau élastique étirable étant assujettie au ruban de fixation (10) au moins à ses deux extrémités (32a, 32b), pour relier une zone du ruban de fixation (10) qui est plus longue que la zone de ruban élastique étirable (32), caractérisé en ce que ladite zone du ruban de fixation (10), qui est plus longue que la zone de ruban élastique étirable (32), est munie d'un pliage en forme de zigzag qui s'étend latéralement par rapport à la longueur du ruban de fixation.

2. Ruban de fixation selon la revendication 1, caractérisé en ce que les extrémités (32a, 32b) de la zone Je ruban élastique (32) sont assujetties au ruban de fixation (10) par un adhésif.

3. Ruban de fixation selon la revendication 1, caractérisé en ce que les extrémités (32a, 32b) de la zone de ruban élastique (32) sont assujetties au ruban de fixation (10) par une réunion hermétique comprenant une réunion hermétique par chaleur et une réunion hermétique par ultrasons.

4. Ruban de fixation selon l'une des revendications 1 à 3, caractérisé en ce que le moyen de fixation (22) est formé par une couche adhésive sur une surface de l'autre partie d'extrémité (20) du ruban de fixation.

5. Ruban de fixation selon l'une des revendications 1 à 3, caractérisé en ce que le moyen de fixation (22) est formé par un moyen de fixation mécanique comprenant les bandes Velcro, les crochets, les boutons et analogues.

6. Ruban de fixation selon l'une des revendications 1 à 5, caractérisé en ce que la zone de ruban (32) réalisée avec un matériau élastique étirable est fixée au ruban de fixation (10) sur l'entière longueur de la zone de ruban de matériau élastique.

7. Ruban de fixation selon l'une des revendications 1 à 5, caractérisé en ce que la zone de ruban (32) réalisée avec un matériau élastique étirable est assujettie au ruban de fixation (10) dans des intervalles entre les extrémités (32a, 32b) du ruban de matériau élastique.

8. Procédé pour fabriquer un ruban de fixation selon l'une des revendications 1 à 7, caractérisé en ce que la zone de ruban élastique (32) est assujettie au moins au niveau de ses extrémités (32a, 32b) au ruban de fixation afin de relier sa partie centrale, et en ce que le stratifié comprenant le ruban de fixation et la zone de ruban élastique est inséré dans un interstice en forme de zigzag de deux matrices.

9. Procédé pour fabriquer un ruban de fixation selon la revendication 8, caractérisé en ce qu'une nappe allongée (38) destinée à former les rubans de fixation est munie d'un ruban (40) réalisé avec un matériau élastique qui est assujetti à la nappe dans sa direction longitudinale (38) en faisant adhérer au moins les bords latéraux du ruban à une surface de la nappe (38), et en ce que la bande de la nappe formant un stratifié de la nappe et du ruban élastique est roulée en anneau, après quoi les rubans de fixation sont formés en coupant la nappe latéralement.

10. Procédé selon la revendication 8 ou la revendication 9, caractérisé en ce que la zone de ruban de matériau élastique étirable est assujettie au ruban de fixation sur l'entière longueur de la zone de ruban de matériau élastique.

11. Procédé selon la revendication 8 ou la revendication 9, caractérisé en ce que la zone de ruban de matériau élastique étirable est assujettie au ruban de fixation dans des intervalles entre les deux extrémités (32a, 32b) du matériau élastique.

12. Procédé selon l'une des revendications 8 à 11, caractérisé en ce que les matrices destinées à déformer le stratifié du ruban de fixation et de la zone de ruban élastique sont formées par des rouleaux annulaires.
